# EUROPEAN PATENT APPLICATION

(11) **EP 4 145 130 A1**
(43) Date of publication of application: **08.03.2023**
(21) Application number: 21797405.4
(22) Date of filing: 22.04.2021
(51) Int. Cl.: G01N 33/53, G01N 33/543

(54) **BIOLOGICAL VESICLE SURFACE BIOMARKER DETECTION DEVICE**

(30) Priority: 27.04.2020 JP 2020078096
(71) Applicant: National University Corporation Tokyo Medical and Dental University, Bunkyo-ku Tokyo 113-8510 (JP)
(72) Inventor: MIYAHARA Yuji, Tokyo 113-8510 (JP); TABATA Miyuki, Tokyo 113-8510 (JP)
(74) Representative: Plasseraud IP
(86) International application number: PCT/JP2021/016271
(87) International publication number: WO 2021/220928

(57) **Abstract**

An object of the present invention is to provide a method, a device, and a system for detecting or measuring a biological vesicle having a size of 20 microns or smaller, such as a cell, an exosome, or an extracellular vesicle among target substances detected by liquid biopsy, in a body fluid-derived sample and quantitatively analyzing the expression level of a target marker molecule, such as a protein, present on the surface thereof. It was demonstrated that a method for qualitatively or quantitatively analyzing the expression of a plurality of target marker molecules and a device and a system therefor could be provided by capturing a body fluid-derived sample containing a plurality of target marker molecules on a support and detecting or measuring the plurality of target marker molecules using an identification substance, such as an antibody. [Selected Drawing] None

## Description

### Technical Field

The present invention relates to providing a method, a device, and a system for quantitatively analyzing the expression level of a target marker molecule, such as a protein, present on the surface of a biological vesicle to be detected or measured in a body fluid-derived sample.

### Background Art

With the advent of an aging society, people are increasingly more interested in health and longevity, and development of non-restraining and non-invasive or minimally invasive diagnostic methods or diagnostic devices for testing for diseases is anticipated. Under such circumstances, liquid biopsy, which is a diagnostic method using body fluids, is drawing attention as a less invasive diagnostic technique than conventional cytological diagnoses and tissue diagnoses, especially in oncology. Currently, target substances detected by liquid biopsy are primarily cells and nucleic acids that circulate in body fluids, and blood circulating tumor cells (CTCs), blood cell-free DNAs (cfDNAs), blood circulating tumor DNAs (ctDNAs), exosomes, microRNAs (miRNAs), and the like are under investigation.

The testing technique of liquid biopsy is a less invasive method than conventional biopsies involving tissue collection and has various clinical significances in disease screening tests, follow-up examinations, prognosis estimation, support for physicians to decide a treatment strategy, and the like. Given that a pain-free test can be realized, an improved rate of having a cancer screening test, leading to early detection of cancer, is expected, as well as an improved cure rate and a longer healthy life expectancy as a result.

Time-series data obtained by these minimally invasive techniques are analyzed using artificial intelligence (AI) or the like, with an aim to use the data for ultra-early detection of diseases, prognosis monitoring, further understanding of health condition, and the like.

ELISA, FACS, Nanoparticle tracking analysis (NTA), and the like are known as methods for detecting cells, biological vesicles (exosomes or extracellular vesicles), and the like among target substances detected by liquid biopsy. The mainstream methods involve optical detection, and the detecting methods have problems that preparation of a measurement sample is complicated, and that miniaturization of a measuring device is difficult. Liquid biopsy is a less invasive method than conventional biopsies involving tissue collection and has various clinical significances in disease screening tests, follow-up examinations, prognosis estimation, support for physicians to decide a treatment strategy, and the like.

Inventions known so far include a device for a biosensor that solidifies virus or bacteria using an oligosaccharide bound on a support and detects the virus or bacteria selectively and very sensitively and a biosensor therefor (Patent Literature 1) and a method for detecting the presence of a target protein based on a pH change by applying the principle of sandwich ELISA, i.e., solidifying a target protein on a sensor using an antibody bound on a support (Non Patent Literature 1). However, conventional techniques have failed to readily and directly detect a target marker molecule, such as a biomarker on the surface of a biological vesicle contained in a body fluid-derived sample, without pretreatment thereof.

### Citation List

### Patent Literature

Patent Literature 1: WO 2017/126617

### Non Patent Literature

Non Patent Literature 1: Biosensors and Bioelectronics 117 (2018) 175-182

### Summary of Invention

### Technical Problem

An object of the present invention is to provide a method, a device, and a system for detecting or measuring a biological vesicle having a size of 20 microns or smaller, such as a cell, an exosome, or an extracellular vesicle among target substances detected by liquid biopsy, in a body fluid-derived sample and quantitatively analyzing the expression level of a target marker molecule, such as a protein, present on the surface thereof.

### Solution to Problem

To solve the above-described problems, the present inventors demonstrated that a method for qualitatively or quantitatively analyzing the expression of a plurality of target marker molecules and a device and a system therefor could be provided by capturing a body fluid-derived sample containing a plurality of target marker molecules on a support and detecting or measuring the plurality of target marker molecules using an identification substance such as an antibody.

More specifically, this application provides the following embodiments to solve the above-described problems:
[1] A method for detecting or measuring a target marker molecule in a body fluid-derived sample on the basis of a pH change or an optical change, the method comprising
   a step of capturing a body fluid-derived sample containing a target marker molecule on a support,
   a step of binding the captured sample to an identification substance that specifically recognizes the target marker molecule in the sample, and
   a step of detecting or measuring a pH change or an optical change generated by the identification substance bound to the target marker molecule.
[2] The method according to [1], wherein a plurality of target marker molecules are contained in a single body fluid-derived sample.
[3] The method according to [2], wherein a step of binding one kind of identification substance to a single body fluid-derived sample and a step of detecting or measuring a pH change or an optical change resulting therefrom are repeated a plurality of times.
[4] The method according to [3], wherein an enzyme bound to a plurality of identification substances that are used in the steps repeated a plurality of times is one kind of enzyme.
[5] The method according to [2], wherein a step of binding a plurality of the identification substances to a single body fluid-derived sample and then detecting or measuring a pH change or an optical change resulting from each identification substances is repeated a plurality of times.
[6] The method according to [5], wherein a different enzyme is bound to each of a plurality of different identification substances.
[7] The method according to any one of [1] to [6], wherein the identification substance is an antibody that specifically binds to the target marker molecule.
[8] The method according to any one of [1] to [7], wherein the body fluid-derived sample is a cell or a biological vesicle.
[9] The method according to [8], wherein the biological vesicle is an exosome or an extracellular vesicle.
[10] The method according to any one of [1] to [9], wherein the pH change or the optical change is generated by a substrate-enzyme reaction.
[11] A device for detecting or measuring a target marker molecule in a body fluid-derived sample, the device comprising
   a support for capturing the body fluid-derived sample, and
   a semiconductor sensor for detecting or measuring a pH change or an optical change generated by an identification substance that specifically recognizes the target marker molecule in the body fluid-derived sample.
[12] The device according to [11], wherein the semiconductor sensor and the support are in the same structure.
[13] The device according to [11] or [12], wherein the semiconductor sensor is a pH sensor selected from an ISFET, an extended-gate FET, and a floating-gate FET or a photosensor selected from a photodiode, a phototransistor, a CCD, and a CMOS image sensor.
[14] The device according to any one of [11] to [13], wherein the support is selected from a film, a glass, a simple silicone substance, and GaAs.
[15] The device according to any one of [11] to [14], wherein the surface of the support is modified to capture a body fluid-derived sample.
[16] The device according to [15], wherein the modification is performed by applying a surface modifier selected from an antibody, a cationic polymer, an extracellular matrix protein, and a hetero molecule having a lipid and a phosphoric acid group, a phosphonic acid group, or a silanol group and a sulfur-containing self-assembled film.
[17] The device according to any one of [11] to [16], wherein the semiconductor sensor electrically detects or measures a pH change or an optical change generated by a substrate-enzyme reaction.
[18] A method for modifying a support for capturing a sample in a body fluid by applying a surface modifier selected from an antibody, a cationic polymer, an extracellular matrix protein, and a hetero molecule having a lipid and a phosphoric acid group, a phosphonic acid group, or a silanol group and a sulfur-containing self-assembled film on the surface of the support.
[19] The method according to [18], for selectively capturing a cell or a biological vesicle in a body fluid.
[20] The method according to [19], wherein the biological vesicle is an exosome or an extracellular vesicle.
[21] A substance for capturing a body fluid-derived sample on a support, the substance is composed of (1) a site to bind to a body fluid-derived sample and (2) a site to bind to a support material.
[22] The substance according to [21], wherein the body fluid-derived sample is a cell or a biological vesicle.
[23] The substance according to [22], wherein (1) the site bind to a body fluid-derived sample is a hydrocarbon chain or a long-chain fatty acid that binds to the lipid membrane of a cell or a biological vesicle.
[24] The substance according to [23], which is a hydrocarbon chain or a long-chain fatty acid that has four or more carbon atoms.
[25] The substance according to any one of [21] to [24], wherein (2) the site to bind to a support material is composed of a silane compound, a phosphoric acid group, a phosphonic acid group, a thiol group, or a disulfide group.
[26] The substance according to [21], wherein the substance is a compound represented by the following: 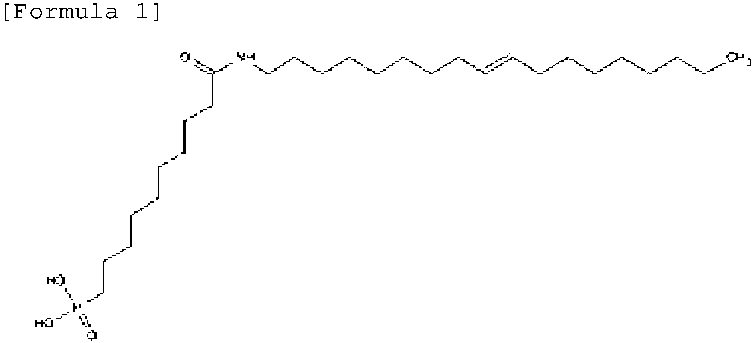
[27] A method for analyzing the expression level of a target marker molecule, the method comprising
   capturing a single body fluid-derived sample on each of a plurality of sensing regions of a semiconductor sensor in a device,
   binding an identification substance that specifically recognizes a target marker molecule in the sample,
   inducing a pH change or an optical change in the vicinity of the sensing region by introducing a substrate,
   digitally counting the number of body fluid-derived samples with a high expression level of the target marker molecule based on the pH change or the optical change, and
   counting the number of semiconductor sensors that have captured body fluid-derived samples with a high expression level of the target marker molecule.

### Advantageous Effects of Invention

Since a body fluid-derived sample holding a plurality of target marker molecules on the surface thereof is solidified on a support with the detecting or measuring technique of the present invention, the target marker molecules in the body fluid-derived sample can be detected or measured by capturing a plurality of target marker molecules on one support as a result, generating a pH change or an optical change individually for each of the target marker molecules, and detecting or measuring these changes. Additionally, by capturing one biological vesicle on one sensor on a 1:1 basis and integrating such sensors into a substrate at high density, the number of biological vesicles with a high expression level of the target marker molecule can be counted and the proportions of biological vesicles with a high expression level of the target marker molecule and biological vesicles with a normal expression level can be obtained with high precision. The quantitativity and efficiency of liquid biopsy using a body fluid-derived sample can be improved substantially by using this technique or device.

### Brief Description of Drawings

[Figure 1] Figure 1 shows one example of the configuration of a device for detecting or measuring a target protein contained in a body fluid-derived sample of the present invention.
[Figure 2] Figure 2 shows an overview of the structure of an ion-sensitive field effect transistor (ISFET) used in experiments.
[Figure 3] Figure 3 shows chemical modification by a molecule having an oleyl group and capture of a biological vesicle.
[Figure 4] Figure 4 shows NMR spectra of synthesized molecules containing an oleyl group.
[Figure 5] Figure 5 shows results of mass spectrometry of synthesized molecules containing an oleyl group.
[Figure 6] Figure 6 shows a sensing region before and after the capture of BT474 cells and pH response characteristics.
[Figure 7] Figure 7 shows visualization of HER2 expression on the surface of BT474 cells. The bar in the figure represents 50 µm.
[Figure 8] Figure 8 shows the configuration of a device for detecting or measuring a biological vesicle surface biomarker using an ISFET and urease.
[Figure 9] Figure 9 shows detection or measurement of a biological vesicle surface marker based on a pH change.
[Figure 10] Figure 10 shows a response of an ISFET to urea without a captured cell.
[Figure 11] Figure 11 shows differences between the response of the device for detecting or measuring a biological vesicle surface biomarker obtained in Figure 9 and the response of the ISFET without a captured cell obtained in Figure 10.
[Figure 12] Figure 12 shows the configuration of a device for detecting or measuring a biological vesicle surface biomarker using glucose oxidase by binding a cell as a biological vesicle to an ISFET.
[Figure 13] Figure 13 shows that the difference between output potentials is a signal reflecting the expression level of a biomarker (EGFR) when the biological vesicle surface biomarker is detected or measured using glucose oxidase.
[Figure 14] Figure 14 shows that the size of a potential change reflects the expression level of a biomarker (EGFR) on the cell membrane surface.

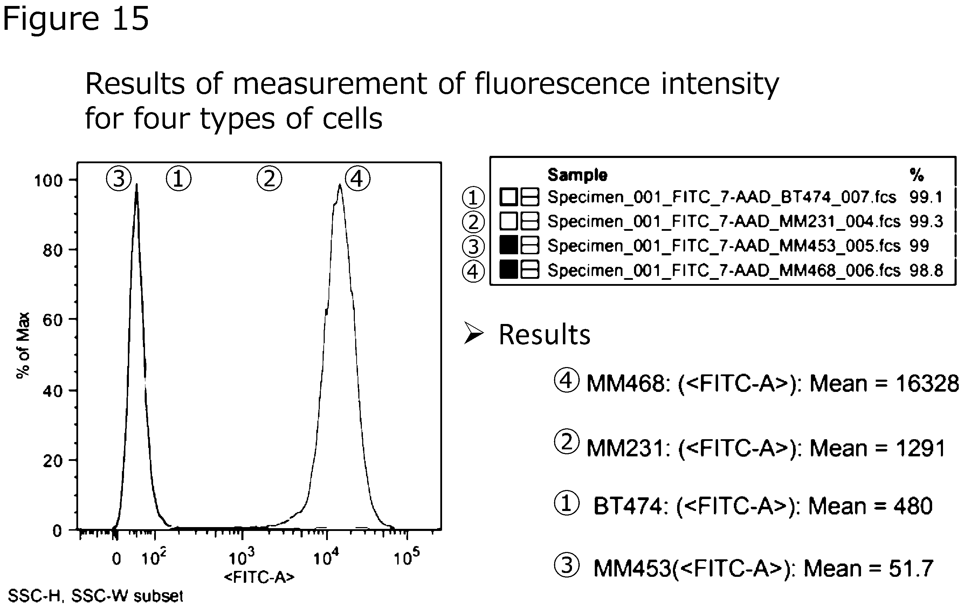

[Figure 15] Figure 15 shows measurements of the intensity of fluorescence using a fluorescence-labeled anti-human EGFR antibody bound to a biomarker (EGFR) on each cell surface by FACS.
[Figure 16] Figure 16 shows correlation between the result of measurement using an ISFET and the result of measurement by FACS about the expression level of a biomarker (EGFR) on the cell membrane surface.
[Figure 17] Figure 17 is a schematic view showing an anti-CD31 antibody-containing magnetic bead, anti-CD105 antibody-containing primary antibody-secondary antibody-enzyme complexes, and a complex of these and MVs.
[Figure 18] Figure 18 shows the relationship between the output potential change of an ISFET and the glucose concentration when MVs were captured by an antibody-containing magnetic bead and a magnet.
[Figure 19] Figure 19 is a schematic view showing immobilization of a molecule having an oleyl group on the gate surface of an ISFET and capture of an MV in the further vicinity of the gate portion of the ISFET.
[Figure 20] Figure 20 shows that the expression level of a protein on a biological vesicle captured by an oleyl group was measured on the basis of the relationship between a change in the ISFET output potential and the glucose concentration.

### Description of Embodiments

### <Testing by liquid biopsy>

The present invention is an invention with an object of substantially expanding the scope of target substances detected by liquid biopsy. Since target substances detected by liquid biopsy have been limited to specific soluble proteins so far, and that a plurality of types of proteins cannot be solidified on a support simultaneously, a plurality of types of proteins cannot be detected or measured from one sample.

In contrast, since a body fluid-derived sample holds a plurality of target marker molecules on the surface thereof, and the body fluid-derived sample is solidified on a support with the detecting or measuring technique of the present invention, this technique has an advantage that a plurality of target marker molecules can be captured on one support as a result. A pH change or an optical change is individually generated for each of the plurality of target marker molecules captured on the support, and a plurality of target marker molecules held by one body fluid-derived sample can be detected or measured simultaneously or sequentially by detecting or measuring the changes simultaneously or sequentially.

Further, by capturing one biological vesicle on one sensor on a 1:1 basis and integrating such sensors in a substrate at high density, the number of biological vesicles with a high expression level of the target marker molecule can be counted and the proportions of biological vesicles with a high expression level of the target marker molecule and biological vesicles with a normal expression level can be obtained with high precision. The quantitativity and efficiency of liquid biopsy using a body fluid-derived sample can be improved substantially by using this technique or device.

### <Method for detecting or measuring a target marker molecule in a body fluid-derived sample>

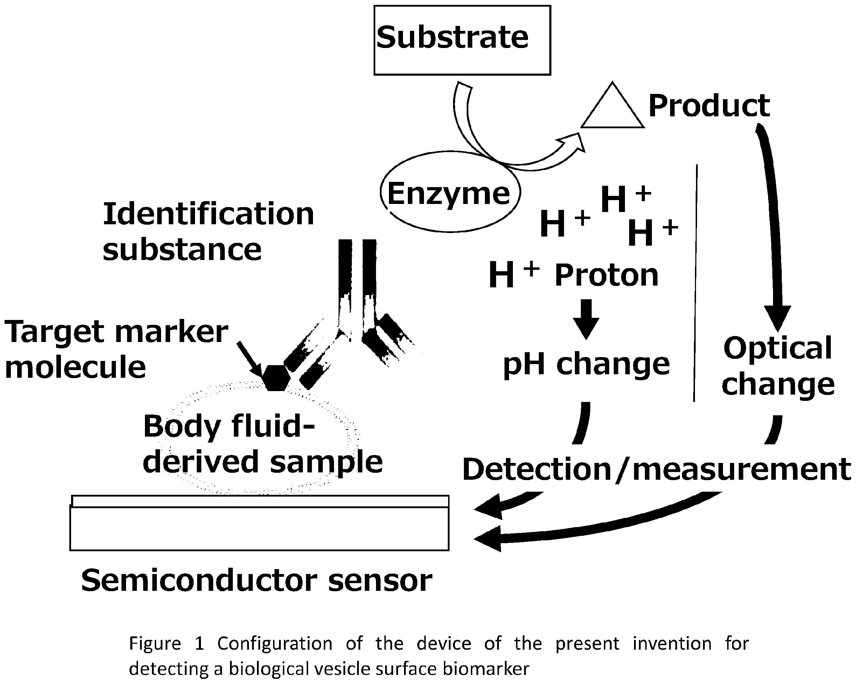

Figure 1 shows one example of the configuration of a system for detecting or measuring a target marker molecule present on the surface of a body fluid-derived sample as one embodiment of the present invention. In one embodiment of the present invention using this detecting or measuring system, a body fluid-derived sample containing a target marker molecule (e.g., a biomarker protein) is captured on a support containing a semiconductor sensor (a sensing region), and an identification substance that specifically recognizes the target marker molecule present on the surface of the body fluid-derived sample is allowed to react to bind specifically to the target marker molecule. Figure 1 shows an antibody as an example of the identification substance. The target marker molecule in the body fluid-derived sample can be detected or measured by detecting or measuring a pH change or an optical change resulting from formation of an identification substance-enzyme complex by binding this identification substance directly or indirectly to an enzyme.

In the present invention, the term "detection" refers to qualitatively finding the presence or absence of a target marker molecule, and the term "measurement" refers to quantifying the abundance of a target marker molecule. Since a pH change or an optical change is generated by an identification substance depending on the amount of a target marker molecule in the method of the present invention, detection or measurement can be performed depending on the sensitivity of a semiconductor sensor.

According to the method of the present invention, the expression levels of target marker molecules on the biological vesicles surface can be quantitatively measured. In addition, the sensors of the present invention can be integrated in a substrate having a predetermined size at high density by manufacturing them with a semiconductor technique. Therefore, by capturing one biological vesicle on one sensor on a 1:1 basis and setting a predetermined threshold for a pH change or an optical change, the number of biological vesicles with an expression level of a target marker molecule being not lower than the predetermined value can be counted. Various biological vesicles are contained in actual body fluids, and it is known that both biological vesicles with a high expression level of a target marker molecule and biological vesicles with a low expression level thereof coexist. By using the method of the present invention, the number of biological vesicles with a high expression level of a target marker molecule and the number of biological vesicles with a low expression level thereof can be individually counted, and their proportions can be calculated. The proportion of biological vesicles with a high expression level of a target marker molecule among biological vesicles in body fluids can serve as an important measure to assess the degree of disease progression or treatment effect, as well as a measure of ultra-early diagnosis of a disease.

The method and the device of the present invention detect or measure target marker molecules present on the surface of a body fluid-derived sample captured on the surface of a support composed of a semiconductor sensor (a sensing region). A plurality of target marker molecules may is present on the surface of a single body fluid-derived sample, and a single or a plurality of target marker molecules may be detected or measured. When, among a large number of target marker molecules present on the surface of a single body fluid-derived sample, only a single specific target marker molecule is to be detected or measured, it is sufficient to use an identification substance that can bind specifically to the target marker molecule. Meanwhile, when, among a large number of target marker molecules present on the surface of a single body fluid-derived sample, a plurality of target marker molecules are to be detected or measured, it is sufficient to use a plurality of different identification substances that can bind specifically to the respective target marker molecules.

In the present invention, an identification substance can be bound to the surface of a body fluid-derived sample depending on the abundance of a target marker molecule on the surface of the body fluid-derived sample. In other words, a larger amount of the identification substance can be bound to the surface of a body fluid-derived sample with a high expression level of the target marker molecule compared with a body fluid-derived sample with a low expression level. As a result, a pH change or an optical change in proportion to the abundance of the target marker molecule on the surface of a body fluid-derived sample will be generated before and after adding a substance for measuring the amount of the identification substance.

Any body fluid-derived samples may be used as long as they are samples derived from body fluids, which can be collected from organisms, such as, for example, blood, urine, cerebrospinal fluid, and saliva. In the present invention, blood is preferred as a body fluid.

In the present invention, among body fluid-derived samples contained in body fluids, cells or biological vesicles are preferably used as body fluid-derived samples because they can hold a plurality of types of target marker molecules. The term "biological vesicles" used herein refers to exosomes and extracellular vesicles.

As target marker molecules to be detected or measured, proteins, sugar chains, peptides, and the like present on the surface of a body fluid-derived sample can be used. Marker molecules known to be related to a disease or a clinical condition, marker molecules that change in conjunction with a treatment effect as a result of treatment of a disease, and the like can be used as these target marker molecules. For example, proteins known as biomarker proteins for diseases and prognoses, such as HER2, EpCAM, CD9, CD63, CD147, EPS8, cytokeratin, and EGFR, can be selected as target marker molecules present on the surface of a body fluid-derived sample.

In the present invention, the above-described identification substance for detecting or measuring a target marker molecule that can be used most commonly is an antibody when the target marker molecule is a target protein, and an antibody or lectin when the target marker molecule is a sugar chain.

When an antibody is used as an identification substance for a target marker molecule, the target marker molecule can be specifically detected or measured by using a primary antibody binding specifically to the target marker molecule and a secondary antibody, which binds specifically to the primary antibody and then forms a complex with an enzyme. Although the primary antibody used in this step binds specifically to a specific target marker molecule, it needs to be distinguished from other primary antibodies and detected or measured in subsequent steps. Therefore, each primary antibody used need to be derived from a different animal species (for example, an antibody derived from a mouse, a rat, a rabbit, a guinea pig, or a goat).

A secondary antibody needs to be selected depending on the type of animal species from which the primary antibody is derived and the type of the antibody (e.g., IgG, IgM). For example, if the primary antibody is a mouse-derived IgG, an anti-mouse IgG antibody needs to be used as a secondary antibody. If the primary antibody is a goat-derived IgG, an anti-goat IgG antibody needs to be used as a secondary antibody. This technique is a technique known to those skilled in the technical field.

In the present invention, an enzyme that generates or consumes proton (H+) by a substrate-enzyme reaction when a substrate is introduced, resulting in a pH change in a solution, or an enzyme whose product of a substrate-enzyme reaction generates an optical change can be used as an enzyme to form a complex with an identification substance for detecting or measuring a target marker molecule. With this technique, a target marker molecule can be detected or measured by measuring a pH change or an optical change after the introduction of a substrate based on the pH or optical intensity before the introduction of a substrate.

When an enzyme that generates or consumes proton as a result of a reaction with a substrate is selected as the above-described enzyme, and an enzymatic reaction is allowed to occur by introducing a substrate, the amount of proton changes in the vicinity of a target marker molecule depending on the expression level of the target marker molecule in a body fluid-derived sample, and the pH changes depending on the change. More specifically, when an enzymatic reaction is allowed to occur by introducing a substrate, the pH changes dependently on the expression level of a target marker molecule on the body fluid-derived sample surface, and this pH change is converted to an electrical signal and detected or measured by a semiconductor sensor such as a pH sensor. The expression of the target marker molecule by detecting or measuring this electrical signal can be qualitatively and quantitatively analyzed.

Enzymes such as, for example, urease, glucose oxidase, penicillinase, glucose dehydrogenase, acetylcholine esterase, and creatinine deiminase can be used as an enzyme that generates or consumes proton (H+) as a result of a reaction with a substrate. Proton is generated or consumed by allowing the respective substrates of the above-mentioned enzymes, such as urea, glucose, penicillin, glucose, acetylcholine, and creatinine, to react, and thus the pH in the vicinity of the reaction field can be changed.

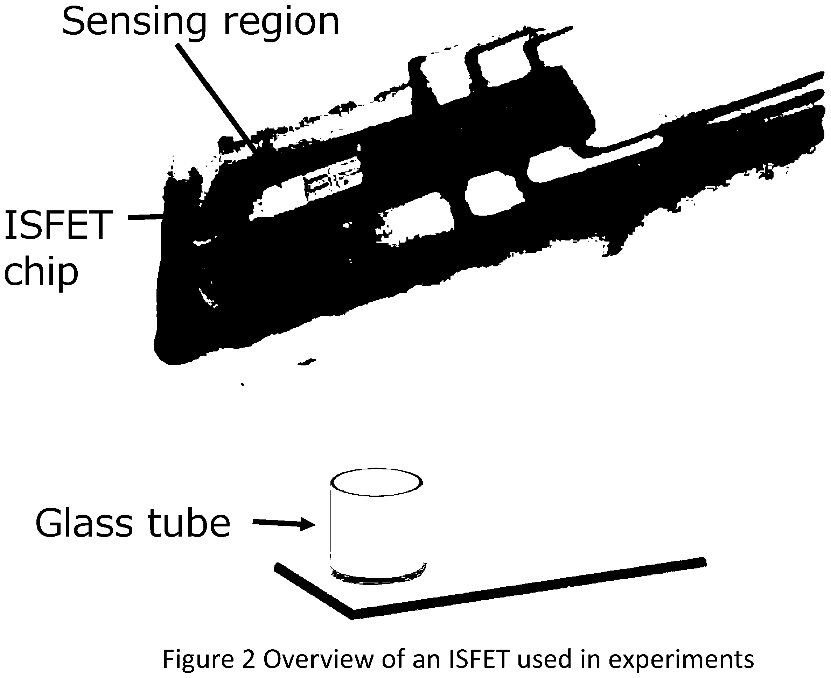

A pH sensor is a sensor that detects or measures a pH difference between a reference electrode and a measuring electrode and converts it to an electrical signal. Several types of pH sensors, such as glass electrodes, metal oxide electrodes, optical fiber pH sensors, ion-sensitive field effect transistors (ISFETs, see Figure 2), extended-gate FETs, and floating-gate FETs, have been developed, and any of these may be used. When an ISFET is used as a pH sensor, one body fluid-derived sample can be captured on the gate (a sensing region) of one transistor by optimizing the gate portion of the ISFET, i.e., the sensing region (see Figure 2), according to the size of a body fluid-derived sample, such as a cell or a biological vesicle, to be captured. By detecting or measuring a pH change generated by introducing a substrate in this state, expression of a target protein on the surface of a single biological vesicle can be detected or measured.

As an alternative method, when an enzyme that generates an optical change as a result of a reaction with a substrate (for example, an enzyme that emits luminescence) is selected as the above-described enzyme, and an enzymatic reaction is allowed to occur by introducing a substrate, the amount of a product that generates an optical change changes dependently on the expression level of a target marker molecule on the surface of a body fluid-derived sample, and the optical change occurs in the vicinity of the body fluid-derived sample. This optical change is converted to an electrical signal using a semiconductor sensor, such as photosensor, and detected or measured. Expression of the target marker molecule can be qualitatively or quantitatively analyzed by detecting or measuring this electrical signal.

For example, enzymes such as alkaline phosphatase (ALP) and horseradish peroxidase (HRP) can be used as an enzyme that generates an optical change as a result of a reaction with a substrate. The reaction product can generate an optical change in the vicinity of the reaction field by allowing dioxetane chemical light-emitting substrates, such as, for example, 3-(2'-spiroadamantane)4-methoxy-4-(3"-phosphoryloxy)phenyl-1,2-dioxetane (AMPPD), CSPD (registered trade name), and CDP-Star^{™}, as chemical light-emitting substrates for ALP or tetramethyl-benzidine (TMB) and luminol chemical light-emitting substrates as chemical light-emitting substrates for HRP to react as substrates for the above-mentioned enzymes.

A photosensor refers to a sensor that senses light and converts it to an electrical signal. For example, several types of photosensors, such as photodiodes, phototransistors, CCDs, and CMOS image sensors, have been developed, and any of these may be used in the present invention. For example, a phototransistor can capture one body fluid-derived sample on the gate (a sensing region) of one transistor by optimizing the sensing region according to the size of the body fluid-derived sample, such as a cell or a biological vesicle, to be captured. By detecting or measuring an optical change generated by introducing a substrate in this state, expression of a target protein on the surface of a single biological vesicle can be detected or measured.

When a plurality of target marker molecules are to be detected or measured, this can be done by repeating a plurality of times a step of capturing a body fluid-derived sample on the sensing region of a semiconductor sensor, such as a pH sensor or a phototransistor, and then binding an identification substance to the single body fluid-derived sample and a step of detecting or measuring a pH change or an optical change, or by repeating a plurality of times a step of binding a plurality of identification substances to a single body fluid-derived sample and then measuring a pH change or an optical change.

In the above methods, when a step of binding an identification substance and a step of detecting or measuring a pH change or an optical change are repeated a plurality of times, one kind of enzyme may be bound to a plurality of identification substances (e.g., antibodies) used in a plurality of steps.

For example, when a plurality of target marker molecules are to be detected or measured by repeating a plurality of times a step of binding an identification substance to a single body fluid-derived sample using a primary antibody-secondary antibody-enzyme complex as an identification substance and a step of detecting or measuring a pH change or an optical change, this may be done by performing the following for a single body fluid-derived sample captured on a support:
· performing a step of binding a primary antibody against a first target marker molecule and a step of measuring a pH change or an optical change,
· performing a step of binding a primary antibody against a second target marker molecule and a step of measuring a pH change or an optical change, and
· repeating this step a plurality of times for a third target marker molecule, a fourth target marker molecule, and so forth.

Or, the method is characterized in that, when a plurality of identification substances are bound to a single body fluid-derived sample, and then a step of measuring a pH change or and an optical change is repeated a plurality of times, different enzymes are bound to a plurality of different identification substances.

For example, when a primary antibody-secondary antibody-enzyme complex is used as an identification substance, a plurality of target marker molecules are to be detected or measured, and the above-mentioned plurality of different identification substances are bound specifically to a plurality of target marker molecules on the surface of a single body fluid-derived sample simultaneously, this may be done by performing the following:
- a step of measuring a pH change or an optical change for a first target marker molecule,
- a step of measuring a pH change or an optical change for a second target marker molecule, and
- performing this step a plurality of times for a third target marker molecule, a fourth target marker molecule, and so forth
after binding a plurality of primary antibodies.

In both methods, expression of the first target marker molecule binding specifically to the first identification substance can be qualitatively or quantitatively examined by detecting or measuring a pH change or an optical change after introducing the substrate of the first enzyme bound to the first identification substance to allow an enzymatic reaction to occur. Additionally, expression of the second target marker molecule binding specifically to the second primary antibody can be qualitatively or quantitatively examined by detecting or measuring a pH change or an optical change after introducing the substrate of the second enzyme bound directly or indirectly to the second identification substance to allow an enzymatic reaction to occur. Thus, a plurality of target marker molecules present on the surface of a single body fluid-derived sample can be sequentially analyzed by measuring a pH change after introducing the substrates of enzymes bound to the primary antibody sequentially into the reaction field to allow an enzymatic reaction to occur.

### <Device for detecting or measuring a target marker molecule in a body fluid-derived sample>

In another embodiment, the present invention provides a detecting or measuring device for realizing the system for detecting or measuring a target marker molecule present on the surface of a body fluid-derived sample shown in Figure 1. This device is provided as a device comprising a support for capturing a body fluid-derived sample and a semiconductor sensor for measuring a pH change or an optical change generated by an identification substance that specifically recognizes a target marker molecule in the body fluid-derived sample.

This device can detect or measure a target marker molecule in the body fluid-derived sample by binding an enzyme directly or indirectly to a target marker molecule present on the surface of a body fluid-derived sample captured on the above-described support and converting a pH change or an optical change resulting from the addition of a substrate corresponding to the enzyme to an electrical signal to detect or measure it.

As the semiconductor sensors held in this device, pH sensors, such as glass electrodes, metal oxide electrodes, optical fiber pH sensors, ion-sensitive field effect transistors (ISFETs, see Figure 2), extended-gate FETs, and floating-gate FETs, can be used to measure a pH change, and photosensors, such as photodiodes, phototransistors, CCDs, and CMOS image sensors, can be used to measure an optical change.

Since a pH change or an optical change generated in response to a target marker molecule present on the surface of a body fluid-derived sample is small in the system of the present invention, a support for capturing a body fluid-derived sample and a semiconductor sensor for detecting or measuring a pH change or an optical change are preferably located as closely to each other as possible to increase the efficiency of detecting or measuring the pH change or optical change. When they are located most closely, the surface of the semiconductor sensor can be used as a semiconductor sensor, and in this case, the support and the semiconductor sensor are in the same structure. In other words, since a component used as a semiconductor sensor itself is used as a support when a support and a semiconductor sensor are in the same structure as described above, the support is selected from a film, a glass, an ISFET, an extended-gate FET, a floating-gate FET, and a phototransistor.

The support may be of any material as long as it can capture a body fluid-derived sample, and a metal oxide such as silicon oxide (glass), iridium oxide, or tantalum oxide, a nitride such as silicon nitride, or the like can be used.

### <Integration of sensors>

The semiconductor sensors used in the present invention can be integrated by miniaturizing one unit of the semiconductor sensors. All pH sensors and photosensors which are examples of the semiconductor sensor used in the present invention have been realized in a small size in a millimeter order and a micrometer order, and these small-size semiconductor sensors can be integrated by arranging them on the support so they can function and measuring them.

For example, the ion-sensitive field effect transistors (ISFETs) shown as an example of pH sensors can be integrated and arrayed at high density because they are micrometer-scale sensors produced by microfabrication technology for semiconductor, and the configuration of the present invention can be constructed on each transistor of the high-density arrayed ISFETs. Therefore, a device which contains highly integrated semiconductor sensors can be provided for detecting or measuring a target marker molecule in a body fluid-derived sample.

The size (area) of the sensing region of the above-described semiconductor sensor preferably has a long side in a range of 100 nm to 50 µm and can be optimized depending on the target substance to be measured. For example, when cells are to be detected, the long side is 5 to 50 µm and is preferably adjusted to an optimal size depending on the type of cells. Additionally, to detect exosomes and extracellular vesicles, the sensing region is characterized by having a long side in a range of 100 to 500 nm.

Thus, by capturing a single body fluid-derived sample individually on each of semiconductor sensors (pH sensors or photosensors) integrated at high density, introducing a substrate, and measuring pH or light intensity, the number of body fluid-derived samples with a high expression level of a target marker molecule can be digitally counted, and the expression level of the target marker molecule can be quantitatively analyzed. Specifically:
a method for analyzing the expression level of a target marker molecule can be provided, the method comprising
capturing a single body fluid-derived sample on each of a plurality of sensing regions of a semiconductor sensor in a device,
binding an identification substance that specifically recognizes a target marker molecule in the sample,
inducing a pH change or an optical change in the vicinity of the above-mentioned sensing region by introducing a substrate,
digitally counting the number of body fluid-derived samples with a high expression level of the target marker molecule based on the pH change or the optical change, and
counting the number of semiconductor sensors which have captured body fluid-derived samples with a high expression level of the target marker molecule.

As described above, by capturing one biological vesicle on one sensor on a 1:1 basis and setting a predetermined threshold for a pH change or an optical change, the number of biological vesicles with a higher expression level of a target marker molecule than the predetermined value can be counted. Various biological vesicles are contained in actual body fluids, and it is known that both biological vesicles with a high expression level of a target marker molecule and biological vesicles with a low expression level thereof coexist. By using the method of the present invention, the number of biological vesicles with a high expression level of a target marker molecule and the number of biological vesicles with a low expression level thereof can be individually counted, and their proportions can be calculated. The proportion of biological vesicles with a high expression level of a target marker molecule among biological vesicles in body fluids can serve as an important measure to assess the degree of disease progression or treatment effect, as well as a measure of ultra-early diagnosis of a disease.

### <Modification of the support surface>

Various modifications may be applied on the support surface to capture a body fluid-derived sample, such as a cell or a biological vesicle. For example, modification can be performed by applying a surface modifier selected from an antibody, a cationic polymer such as poly-L-lysine, an extracellular matrix protein such as collagen, fibronectin, and vitronectin, and a hetero molecule having a lipid such as an oleyl group and a phosphoric acid group, a phosphonic acid group, or a silanol group, and a sulfur-containing self-assembled film on the support surface. These modifications can be introduced into the support surface by pre-treating the support surface with a silane coupling agent and then introducing various modifications thereafter.

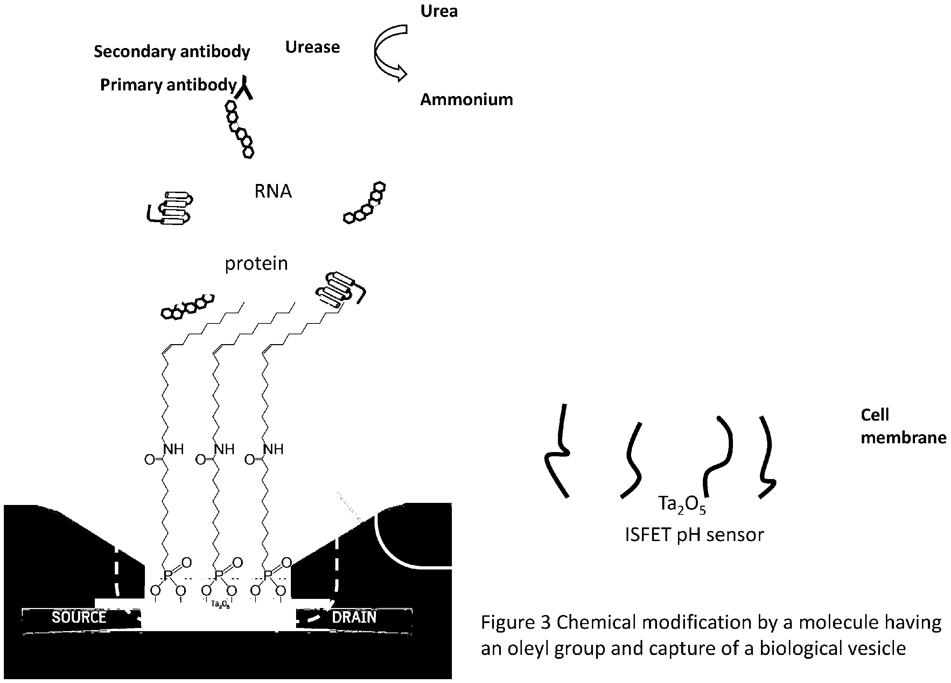

As described above, examples of the surface modifier to capture a body fluid-derived sample on the support include and a hetero molecule having a lipid such as an oleyl group and a phosphoric acid group, a phosphonic acid group, or a silanol group, but this specific configuration can be exemplified as a substance for capturing a body fluid-derived sample on a support composed of (1) a site to bind to a body fluid-derived sample such as a biological vesicle and (2) a site to bind to a support material (Figure 3). When the body fluid-derived sample is a cell or a biological vesicle, (1) the site to bind to a body fluid-derived sample binds to the lipid membrane thereof, and is composed of a hydrocarbon chain or a long-chain fatty acid. The hydrocarbon chain or the long-chain fatty acid used in this embodiment is characterized by preferably having four or more carbon atoms, and, for example, an oleyl group based on a lipid can be used. Further, (2) the site to bind to a support material can bind to a support surface composed of a metal oxide such as silicon oxide (glass), silicon nitride, or tantalum oxide as a material. This (2) site to bind to a support material is composed of an organosilane compound, a phosphoric acid group, a phosphonic acid group, a thiol group, or a disulfide group. When a molecule having the above-described configuration of (1) and (2) is formed on the support, an oleyl group is fused with the lipid membrane of a body fluid-derived sample (e.g., a biological vesicle), so that the body fluid-derived sample can be captured on the support. Additionally, when this molecule is formed on the sensing region of the semiconductor sensor, the body fluid-derived sample can be captured on the sensing region. As an example of the hetero molecules in the present invention, one represented by the following formula: 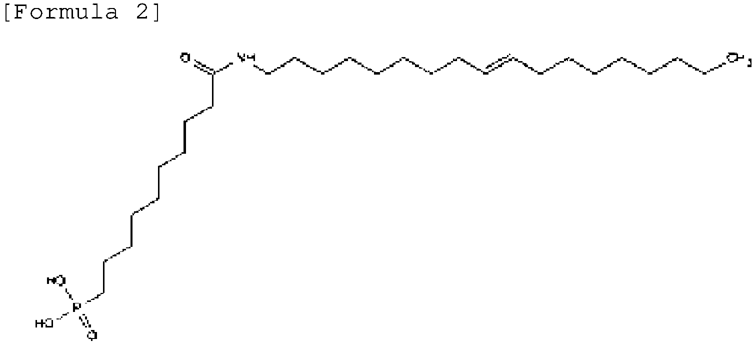 can be used.

Examples of detection and measurement using an enzyme that generates proton (H+) and changes pH dependently on the expression level of a target marker molecule on the surface of a body fluid-derived sample when a pH sensor was used as a semiconductor sensor are shown below. When an enzyme generating an optical change is used, target marker molecules can be similarly detected with the same principle, except that a photosensor is used as a semiconductor sensor.

### Examples

### Example 1: Surface modification of the sensing region

In this example, the configuration of a device using a pH sensor as a semiconductor sensor was investigated.

In this experiment, an ISFET (ISFETCOM Co., Ltd.) shown in Figure 2 was used as a pH sensor. For this pH sensor, tantalum pentoxide (TazOs) was used as a material for a pH sensitive film having a thickness of 40 nm, and a gate having a length of 10 µm and a width of 300 µm served as a sensing region of a hydrogen ion (proton).

A glass tube having an inside diameter of 5 mm was adhered onto the ISFET surface by curing a thermosetting epoxy resin at 120°C for 2 hours, so that this gate region was included. A cell was captured as a body fluid-derived sample into this tube to form a reaction field.

To capture a body fluid-derived sample on the sensing region, which was a gate portion of the ISFET, the TazOs surface was chemically modified. First, the TazOs surface was washed with a mixed solution of 60 µL of NH₄OH (10% v/v) and 140 µL of H₂O₂ (20% v/v) and water, immersed in a poly-L-lysine aqueous solution (0.01% w/v) at room temperature for 10 minutes to adsorb poly-L-lysine to the TazOs surface, then washed with pure water, and dried. Given that poly-L-lysine has a positive electric charge based on an amino group, whereas the surface of a biological vesicle has a negative electric charge, the biological vesicle can be captured on the poly-L-lysine surface by electrostatic interaction. The device thus produced was used in the following examples.

### Example 2: Synthesis of an oleyl group-containing molecule

In this example, an oleyl group-containing molecule used in an embodiment in which a biological vesicle is captured on the surface of a sensor substrate (Figure 3) was synthesized.

In amount of 20 mg of 10-carboxydecylphosphonic acid (10CDPA) (Dojindo) was added to 1 mL of tetrahydrofuran (THF), 35 mg of (4,6-dimethoxy-1,3,5-triazin-2-yl)-4-methylmorpholinium chloride (DMT-MM) (Wako) and 6 µL of oleylamine (Wako) were further added and mixed, and the mixture was left to stand at room temperature for 2 hours to allow a reaction to occur.

An excessive amount of DMT-MM and an excessive amount of oleylamine were removed from this mixture solution, a solid-phase aggregate obtained as a result was dissolved in a 1,4-dioxane solvent, and the solution was cooled with liquid nitrogen for 3 to 4 hours to be lyophilized and pulverized.

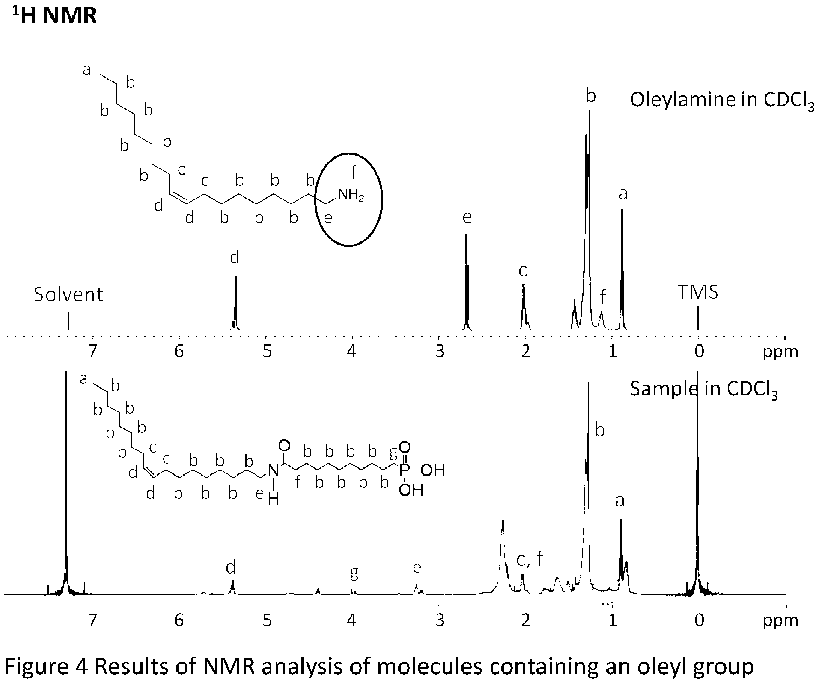

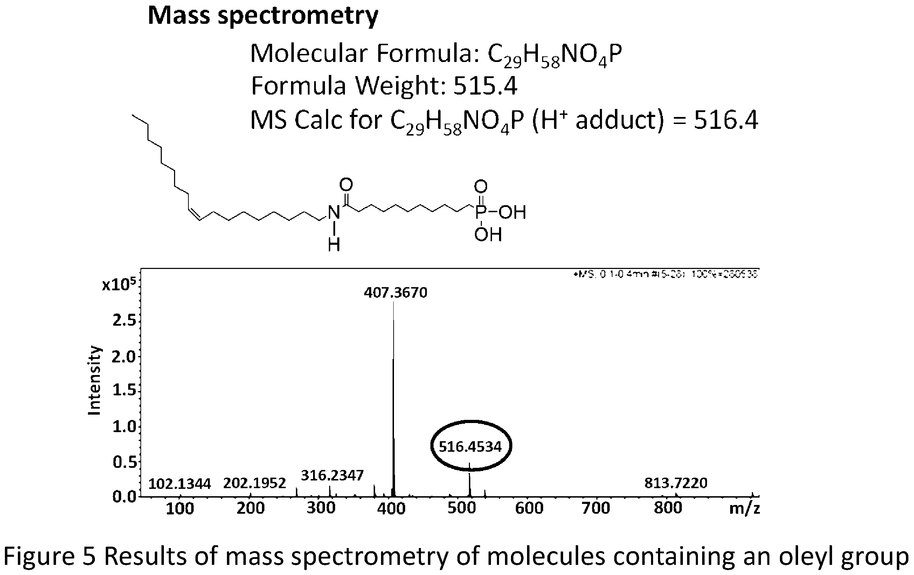

Figure 4 shows the NMR spectra of the oleyl group-containing molecules synthesized by the above-described process. The results confirmed that an amide bond formation reaction was proceeding. Figure 5 shows the results of mass spectrometry of the synthesized oleyl group-containing molecules. The chemical formula of the target substance is C₂₉H₅₈NO₄P, with a molecular weight of 515.4, and the molecular weight of the synthesized compound was 516.45, demonstrating that the target substance represented by the following chemical formula was successfully synthesized.

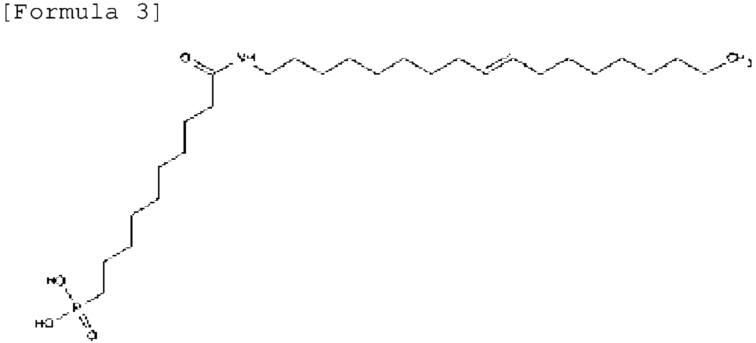

### Example 3: Cell culture

In this example, cells used as examples of body fluid-derived samples and culture thereof were investigated.

As the body fluid-derived samples, human breast cancer cell line BT474 (ATCC No. HTB-20), MCF10A (ATCC No. CRL-10317), and MDA-MB-231 (ATCC No.HTB-26) (American Type Culture Collection (ATCC, Manassas, VA)) were used.

MEGM^{™} Mammary Epithelial Cell Growth Medium (Lonza Walkersville, MD, USA) was used as a culture broth for MCF10A cells, and RPMI 1640 1× (Thermo Fisher Scientific, MA, USA) containing 10% fetal bovine albumin (FBS), penicillin-streptomycin 1× (Wako, JAPAN), and GlutaMax 1× (Thermo Fisher Scientific, MA, USA) was used as a culture broth for other cells.

All the above-mentioned cells were first cultured on a cell culture dish at 37°C in a 5% CO₂ atmosphere. After treated with trypsin, 10⁵ BT474 cells/mL cells were seeded on the sensing region of the ISFET (ISFETCOM Co., Ltd.) subjected to chemical modification with poly-L-lysine or an oleyl group-containing molecule (investigated in Example 1) and cultured overnight at 37°C in a 5% CO₂ atmosphere.

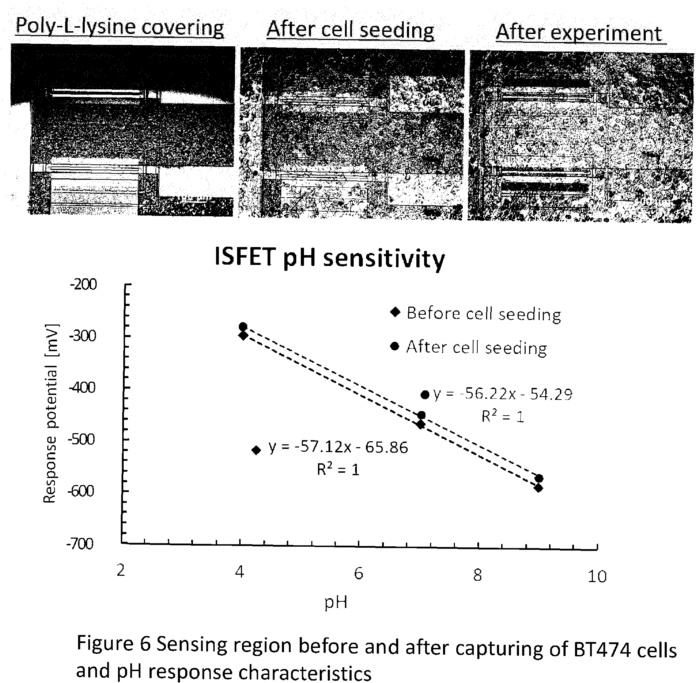

The pH response characteristics of the ISFET were assessed while the cells were being captured on the sensing region of the ISFET. An Ag/AgCl electrode having a salt bridge was provided in the glass tube formed on the sensing region of the ISFET, and the potential response of the ISFET was measured when a buffer solution of pH 4, 7, or 9 was introduced. The results are shown in Figure 6. Even when the TazOs surface of the sensing region was subjected to chemical modification with poly-L-lysine or an oleyl group-containing molecule, and cells were further captured on top thereof and cultured, the pH response characteristics of the ISFET hardly changed, showing that the pH response characteristics such as favorable sensitivity and stability were maintained.

### Example 4: Confirmation of HER2 expression using a confocal fluorescence microscope

In this example, the expression status of HER2, which is used as one of markers for the cell membrane surface test for breast cancer, on BT474 cells was examined using a conventional technique.

BT474 cells were treated with trypsin, seeded on a glass dish, and cultured overnight at 37°C in a 5% CO₂ atmosphere. A paraformaldehyde solution prepared at 4% with a phosphate buffer solution was added dropwise to allow a reaction to occur for 20 minutes, and cells were washed three times with a phosphate buffer solution. Subsequently, to prevent non-specific adsorption, a blocking solution comprising 10% v/v goat normal serum (Vector Laboratories), 0.2% v/v Tween 20 (Nacalai Tesque; 28353-85), and 0.7% v/v glycerol (Wako, Japan) was introduced into the BT474 cell surface to allow a reaction to occur for 1 hour. The solution was further allowed to react with a rabbit anti-HER2 polyclonal antibody (SAB4500789; Sigma) diluted to 1/200 for 1 hour. Cells were washed with a phosphate buffer solution, and an FITC-labeled anti-rabbit antibody (Abcam, Cambridge, UK) diluted to 1/400 was added to allow a reaction to occur for 1 hour.

Subsequently, to stain with fluorescence, cells were allowed to react with 4,6-diamidino-2-phenylindole (DAPI; FUJIFILM Wako Pure Chemical Corporation) diluted with a phosphate buffer solution to 1/1000 for 10 minutes. After cells were washed, expression of HER2 on the cell membrane surface was observed using a confocal fluorescence microscope (Nikon ECLIPSE Ti-E confocal microscope with LU-N series laser unit (Nikon, Tokyo, Japan)).

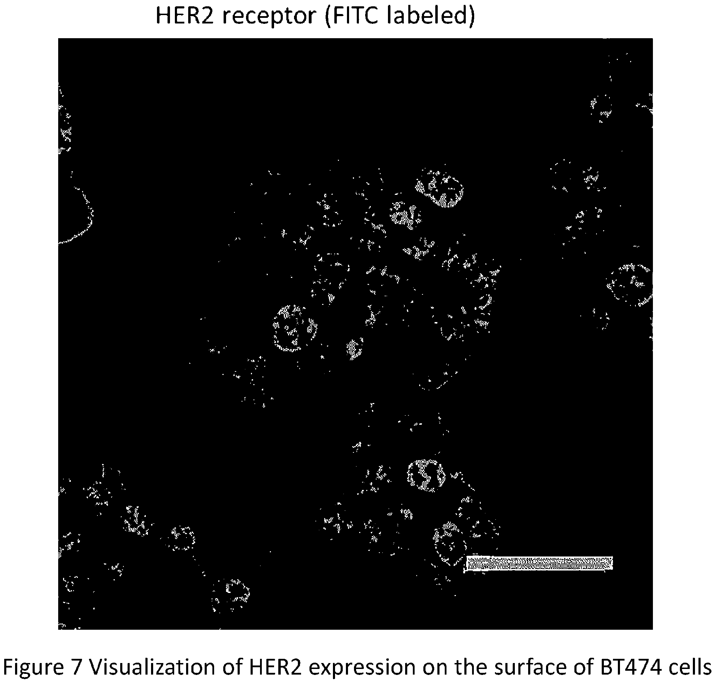

The results are shown in Figure 7 (the bar in the figure represents 50 µm). The results confirmed that HER2 was expressed on the BT474 cell membrane surface.

### Example 5: Detection of HER2 using the device for detecting a target marker molecule on the surface of a body fluid-derived sample of the present invention

In this example, whether HER2 could be detected using the device of the present invention was examined for BT474 cells, for which expression of HER2 on the cell surface thereof had been confirmed.

As in the method described in Example 2, the surface of the sensing region of the ISFET was subjected to chemical modification with poly-L-lysine, and 10⁵ BT474 cells/mL cells were captured and cultured overnight at 37°C in a 5% CO₂ atmosphere, then a paraformaldehyde solution prepared with a phosphate buffer solution at 4% was added dropwise to allow a reaction to occur for 20 minutes, and then cells were washed three times with a phosphate buffer solution. The above-described blocking solution was introduced into the surface of BT474 cells to allow a reaction to occur for 1 hour, and cells were washed and further allowed to react with a rabbit anti-HER2 polyclonal antibody (SAB4500789; Sigma) diluted to 1/200 for 1 hour. After cells were washed, an anti-rabbit antibody bound to urease diluted to 1/1600 was introduced to allow a reaction to occur for 90 minutes to bind to the anti-HER2 polyclonal antibody, and then cells were washed to remove unbound anti-rabbit antibody.

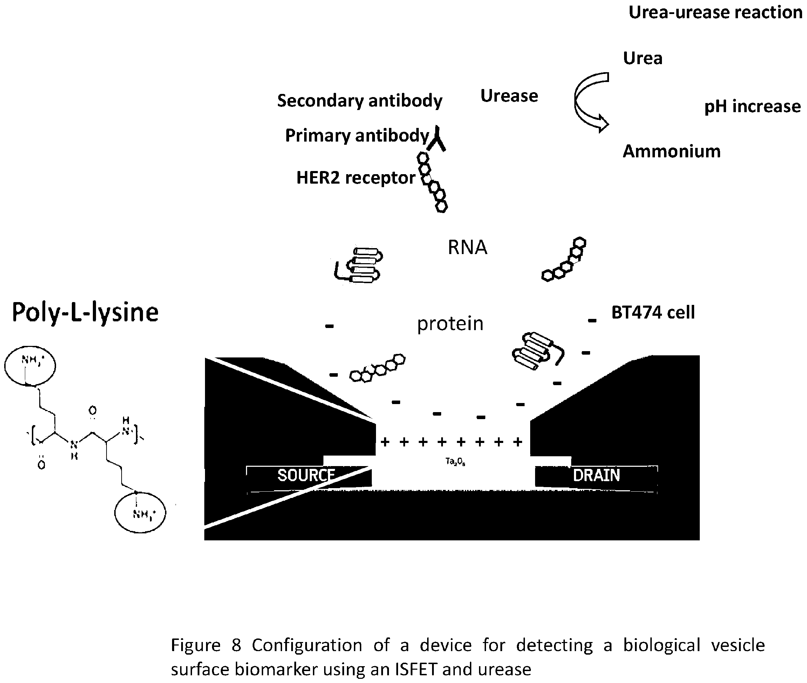

Figure 8 shows the configuration of the device for detecting a target marker molecule on the surface of a body fluid-derived sample, which was constructed by the above processes. The device has the same fundamental concept as that of the present invention shown in Figure 1. In this example, the presence of HER2 on the cell surface was detected or measured on the basis of a pH change by using an ISFET, which is a pH sensor, as a semiconductor sensor, a primary antibody that recognizes HER2 and a secondary antibody that recognizes the primary antibody as identification substances, and urease as an enzyme bound to the secondary antibody.

Urea was introduced into the sensing region of the above-described device for detecting a target marker molecule on the surface of a body fluid-derived sample, urease and urea were allowed to react on the cell surface, and a pH change generated by a proton (H+) resulting from the reaction was monitored using the ISFET. An Ag/AgCl electrode containing 100 mM potassium chloride aqueous solution as an internal liquid was used as a reference electrode and brought into contact with a sample solution via a salt bridge comprising agarose gel.

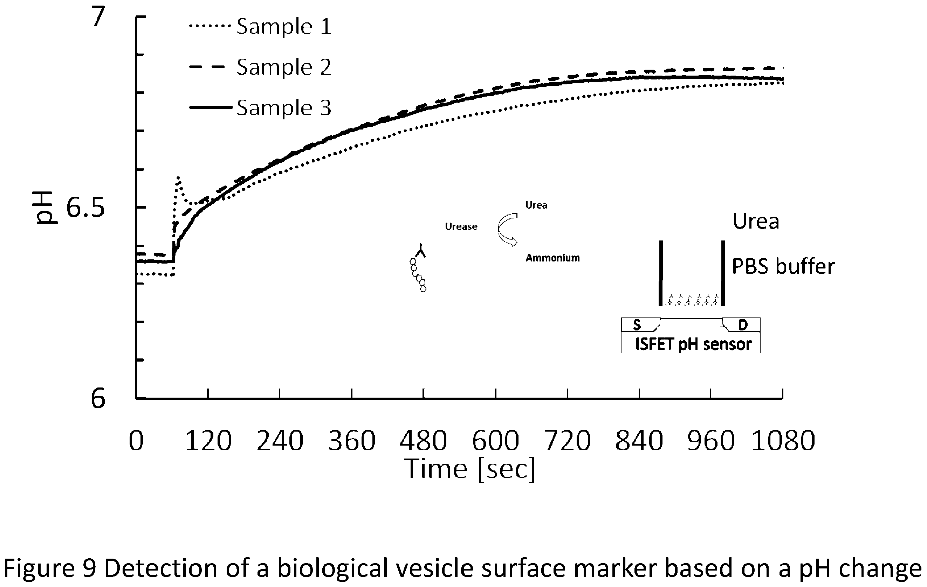

Figure 9 shows the results of responses when a 15 mM urea solution was introduced into the glass tube of three devices for detecting a target marker molecule on the surface of a body fluid-derived sample which have the same configuration. The potential responses converted to pH changes are shown. The responses of the three devices after introduction of the urea solution were gradually changed towards the alkaline side, and a steady state was achieved in approximately 15 minutes. The pH changed from 6.3 to 6.9 by 0.6 in 15 minutes. By the way, the degradation reaction of urea by urease is expressed by the following formula (1):

(NH₂)₂CO + 2H₂O + H⁺ → 2NH₄⁺ + HCO₃⁻ (1)

This formula (1) indicates that a hydrogen ion (H+) is consumed along with progression of the enzymatic reaction of urease. In other words, pH changes towards the alkaline side. Therefore, it is understood that the change towards the alkaline side observed in Figure 9 was a change resulting from the reaction of urease and urea on the cell surface. Since the amount of urease binding to the cell surface reflects the expression level of HER2, it means that the characteristics of the obtained pH change reflect the expression levels of the HER2 on the cell surface. Therefore, the expression level of HER2 can be obtained by measuring the reaction rate of the pH change or the quantity of the pH change.

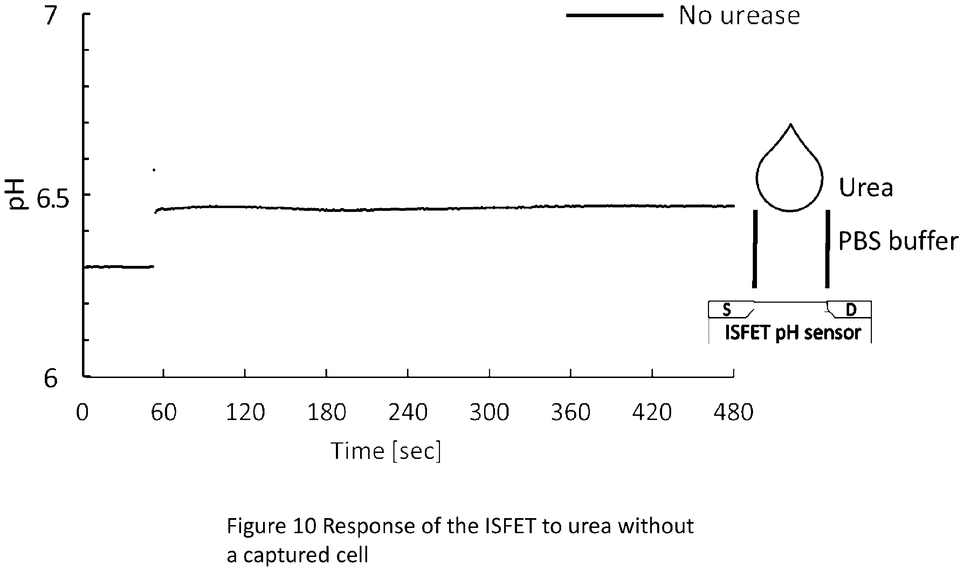

Figure 10 shows responses when a 15 mM urea solution was introduced into the sensing region as described above of the ISFET without a captured BT474 cell as a negative control. After introduction of urea, the pH instantly changed from 6.3 to 6.5 by 0.2 and remained at a constant value thereafter. This change was smaller than the pH change with a captured cell shown in Figure 9, but was expected to be a pH change simply resulting from mixing because the pH of the solution for measurement and the pH of the 15 mM urea solution introduced later were different in the first place.

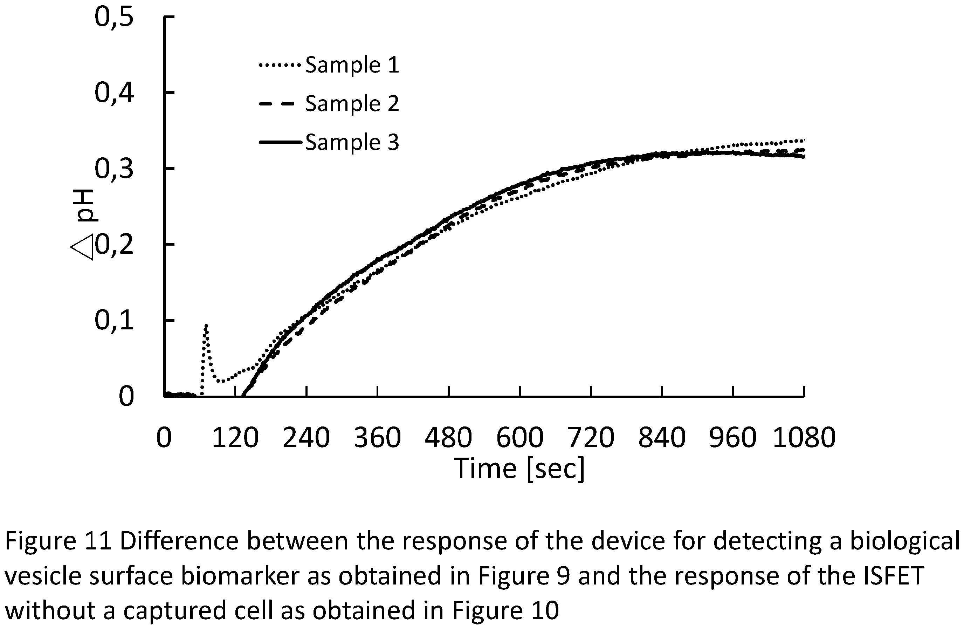

Figure 11 shows differences between the response of the device for detecting a target marker molecule on the surface of a body fluid-derived sample to the 15 mM urea solution obtained in Figure 9 and the response of the ISFET without a captured cell (a negative control) obtained in Figure 10. A background such as a change in the response of the ISFET device caused by a pH change in the whole mixture solution due to the difference in pH of two solutions or temperature difference can be deducted by this differential measurement. As a result, only a pH change reflecting the expression level of HER2 on the cell surface based on the enzyme-substrate reaction can be extracted. It was found that pH had changed by the reaction between urease and urea by approximately 0.35 in 15 minutes, dependently on the expression level of HER2 on the BT474 cell membrane surface. This result indicates that monitoring of increases in pH due to the enzymatic reaction of urease targeting HER2 expressed on the BT474 cell surface was successful.

As described above, it was demonstrated that the device for detecting a target marker molecule on the surface of a body fluid-derived sample of the present invention can monitor HER2, which is a target marker molecule, expressed on the surface of a BT474 cell, which is a body fluid-derived sample, using a pH increase resulting from an enzymatic reaction of urease and can detect or measure the expression level of the target marker molecule. In this example, a cell was used as a body fluid-derived sample, but it was shown that this detection principle could also be applied to other body fluid-derived samples (e.g., an exosome and an extracellular vesicle).

### Example 6: Quantitative detection of the expression level of a protein on the cell surface

This example examined an example of quantitatively detecting differences in the expression level of an epidermal growth factor receptor (EGFR) protein on the surface of a breast cancer cell.

### (6-1) Experiment protocol

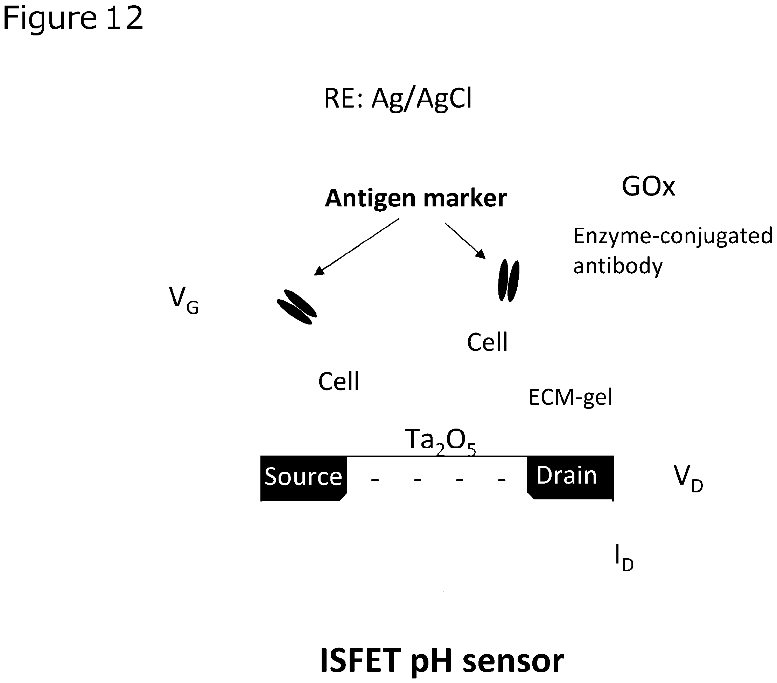

An extracellular matrix (ECM) gel layer for capturing a biological vesicle was formed on the gate insulator film surface of a pH sensitive field effect transistor, and any of four types of established cell lines, BT474, MM231, MM468, and MM453 was individually seeded thereon (see Figure 12).

Specifically, each of BT474, MM231, MM468, and MM453 was cultured beforehand, abrased from a culture dish by trypsin treatment, and then centrifuged at 3000 rpm for 5 minutes to collect cells, and a cell suspension was prepared at a concentration of 1 × 10⁶ cells/mL.

In a volume of 200 µL of cell suspension (containing 0.2 × 10⁶ cells) was seeded into a reaction chamber (a glass ring) formed on the ISFET surface where an ECM gel was formed, and cells were cultured at 37°C in a 5% CO₂ atmosphere for 24 hours. Then, a 2% bovine serum albumin (BSA) solution prepared with a bis-tris propane (BTP) buffer solution was added to the reaction chamber as a blocking solution. A reaction was allowed to occur at 37°C in a 5% CO₂ atmosphere for 1 hour, and cells were washed three times with a BTP buffer solution. By this step, BSA is adsorbed to the cell surface, the ECM gel surface, and the reaction chamber surface, preventing non-specific adsorption.

To form an antibody-enzyme complex on the cell surface depending on the expression level of EGFR, first, an anti-human EGFR antibody (BioLegend Inc.) was added as a primary antibody to allow a reaction to occur at 37°C for 30 minutes. Subsequently, 50 µL of a secondary antibody that had formed a complex with an enzyme glucose oxidase was added to allow a reaction to occur at 37°C for 30 minutes. Finally, the supernatant was discarded, and cells were washed three times with a BTP buffer solution.

### (6-2) Confirmation of correlation between generation potential and the expression level of a cell membrane surface protein (EGFR)

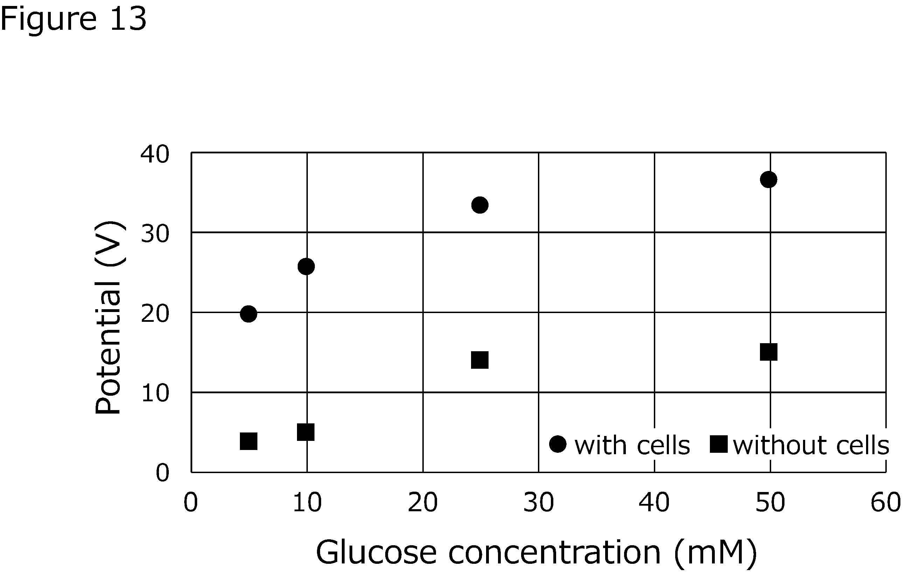

Figure 12 shows a schematic structural view of the cell-immobilized ISFET prepared as described above. As an example, 50 µL of a BTP buffer solution was added to the ISFET reaction chamber in which MM468 cells were immobilized, the output voltage of the ISFET was measured, and each of 5 mM, 10 mM, 25 mM, and 50 mM glucose solutions was added at the timepoint when the output voltage became stable. Changes in the output potential of the cell-immobilized ISFET at this time are shown in Figure 13. The response (■) of the ISFET without a cell immobilized thereon was also shown as a control.

The output potential (●) of the cell-immobilized ISFET increased dependently on the glucose concentration. The increase in the output potential indicates that pH in the vicinity of the gate portion changed towards the acidic side. It appears that this is because the enzyme glucose oxidase bound to EGFR expressed on the cell surface via a primary antibody and a secondary antibody, glucose was degraded by an enzymatic reaction dependently on the glucose concentration to generate gluconic acid, and pH changed locally in the vicinity of the ISFET gate portion (the sensing portion).

The reason why the output potential change tends to show a saturating tendency with a higher glucose concentration is that shortage of supply of dissolved oxygen occurs temporarily in the vicinity of the cell surface in a high glucose concentration region because dissolved oxygen is consumed along with the enzymatic reaction, and the rate of the enzymatic reaction is limited by the concentration of dissolved oxygen.

The response (■) of the ISFET without a cell immobilized thereon was also shown to be dependent on the glucose concentration although the potential change was small. It is considered that this is because a secondary antibody-enzyme complex was non-specifically adsorbed to the ECM gel surface or the like since the primary antibody and the secondary antibody were added to the ISFET surface without a cell, and glucose was degraded by an enzyme by the addition of glucose, resulting in a pH change. It is considered to be a signal of the difference in the output potentials between the cell-immobilized ISFET (●) and ISFET without immobilized cells (■), which reflects the expression level of EGFR. On the basis of this result, the output potential from the secondary antibody-enzyme (glucose oxidase) complex was to be measured at 10 mM glucose concentration in the following example (6-3).

### (6-3) Correlation between the size of a potential change and the expression level of a cell membrane surface protein (EGFR)

Subsequently, the expression levels of EGFR on the cell membrane surface were shown to be different among four types of established cell lines, BT474, MM231, MM468, and MM453.

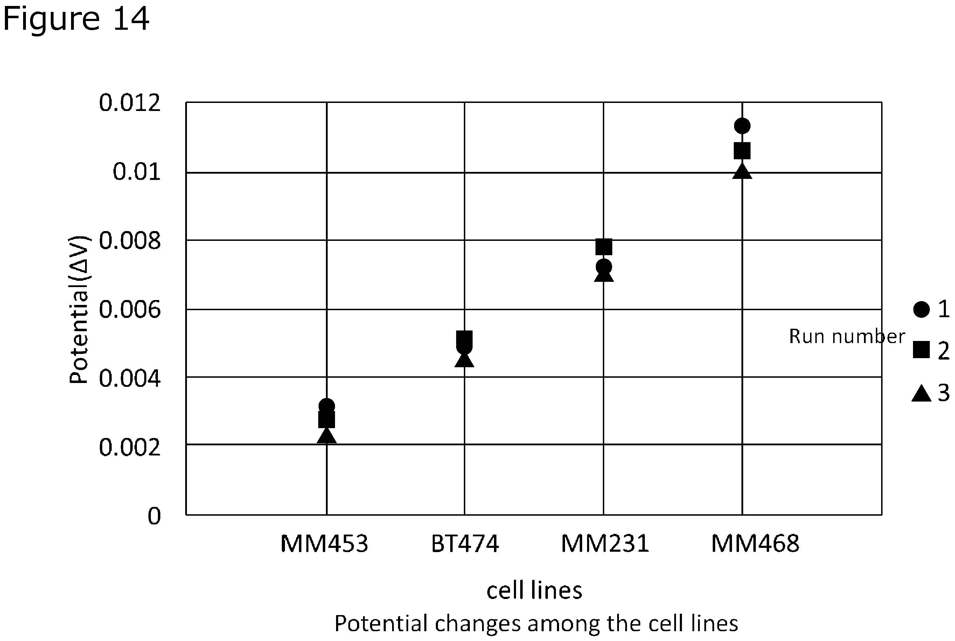

Each of BT474, MM231 and MM453 was captured on the gate surface of the ISFET, an enzyme glucose oxidase was immobilized on the cell membrane surface via a primary antibody and a secondary antibody using the same protocol as used when MM468 cells were captured in the above (6-2), and the response (output potential) upon addition of a 10 mM glucose solution was measured. The results of comparing responses of the ISFET that captured four types of cells (MM453, BT474, MM231, and MM468) are shown Figure 14. The size of the potential change was larger with MM453, BT474, MM231, and MM468 in this order, indicating that the potential change reflects the expression level of EGFR on the cell membrane surface.

To confirm the correlation between the size of potential change and the expression level of EGFR on the cell membrane surface, the expression levels of EGFR on the surface of the above-mentioned four types of cells were analyzed by fluorescence-activated cell sorting (FACS). Cells were stained by binding a fluorescence-labeled anti-human EGFR antibody to the above-mentioned EGFR on the cell surface, and fluorescence intensity was compared. The results of measurement of fluorescence intensity for MM453, BT474, MM231, and MM468 cells are shown in the figure. The results show that the fluorescence intensity, that is, the expression level of EGFR, was higher for MM453, BT474, MM231, and MM468 in the order, which is consistent with the tendency in the measurement using an ISFET.

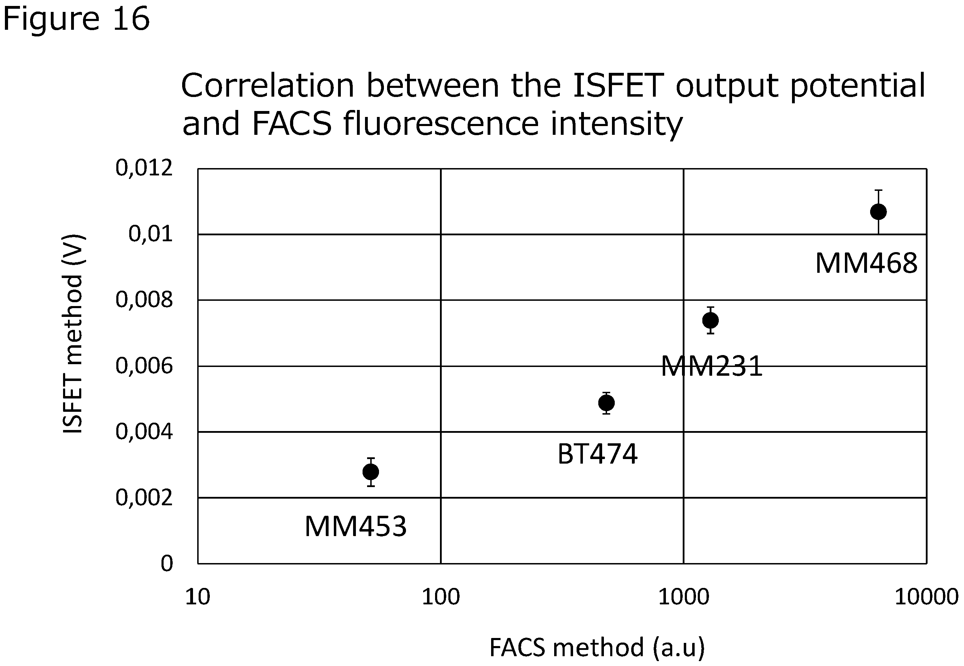

Figure 16 shows the results of examination of the correlation in the expression levels of EGFR on the cell membrane surface of four types of cells MM453, BT474, MM231, and MM468 between the results of measurement using an ISFET (vertical axis) and the results of measurement using FACS (horizontal axis). The results show that there is a favorable correlation between them, demonstrating that the cell-immobilized ISFET of the present invention can quantitatively analyze the expression level of EGFR on the cell membrane surface.

This method can also be applied to cell surface markers other than EGFR by replacing the antibodies. Additionally, a plurality of cell surface markers can be analyzed sequentially while cells are being captured on the ISFET surface by using enzymes other than glucose oxidase, such as, for example, the above-described urease, in combination.

### Example 7: Measurement of the expression level of a protein on a biological vesicle captured by a bead

This example was intended to show that the configuration of the present invention could be applied to not only cells shown in Example 6, but also other biological vesicles.

A human umbilical vein endothelial cell (HUVEC)-derived MV was bound to a magnetic bead, the MV was localized on the gate surface of the ISFET by placing a magnet on the back surface of the ISFET, and the output potential of the ISFET was measured after adding glucose.

Specifically, 100 µL each of an anti-mouse IgG secondary antibody (goat) conjugated with 30 µg/mL enzyme glucose oxidase (Glucose Oxidase Conjugation Kit-Lightning-Link, Goat anti-Mouse IgG H&L; Abcam) and 20 µg/mL anti-human CD105 primary antibody (mouse IgG) (Purified anti-human CD105; BioLegend) were poured into a 1.5 mL tube and mixed with gentle tapping, and then the mixture was left to stand in a refrigerator for 30 minutes. CD105, which is expressed on the surface of an MV as one of numerous surface molecules, was bound to a primary antibody-secondary antibody-enzyme complex.

In a volume of 1 µL of 4 × 10⁵ anti-CD31 antibody-immobilized bead and washed beads/mL (Dynabeads^{™} CD31 Endothelial Cell; Invitrogen) was added, and the mixture was left to stand at 4°C for 30 minutes. Since anti-CD31 antibody is immobilized on the surface of the magnetic bead, an MV is bound on the surface of the magnetic bead. CD31 is platelet endothelial cell adhesion molecule-1 (PECAM-1) and is known as a marker of HUVEC.

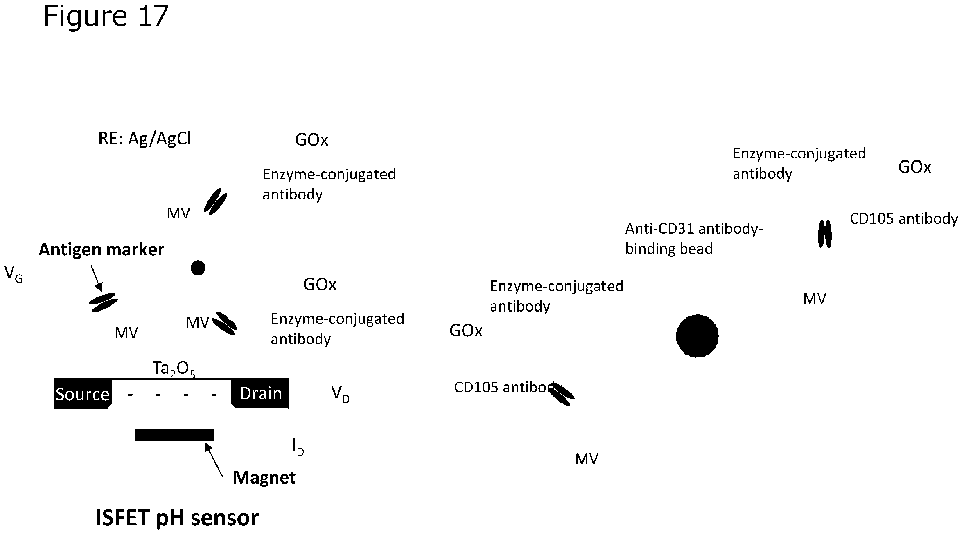

Then, the mixture solution was left to stand on the magnet (DynaMag^{™}-2; Invitrogen) for 2 minutes, beads were collected, and the supernatant was discarded. After removed from the magnet, the beads were washed by adding 100 µL of 1 mM phosphate buffer solution, pipetting the mixture several times, leaving it to stand for 2 minutes again, and discarding the supernatant. This washing step was repeated three times. Figure 17 shows a schematic view of the anti-CD31 antibody-containing magnetic beads prepared as described above, a primary antibody-secondary antibody-enzyme complex containing an anti-CD105 antibody, and a complex of these and an MV.

Subsequently, a neodymium magnet (1 mm × 1 mm × 2 mm) was placed on the back side of the gate position of the ISFET equipped with a chamber. The MV solution prepared by the above-described procedure was added dropwise, and the mixture was left to stand for approximately 20 minutes. ISFET measurement was started, a glucose solution (0 mM, 5 mM, 10 mM, or 25 mM) or a phosphate buffer solution was added, and the potential response was recorded.

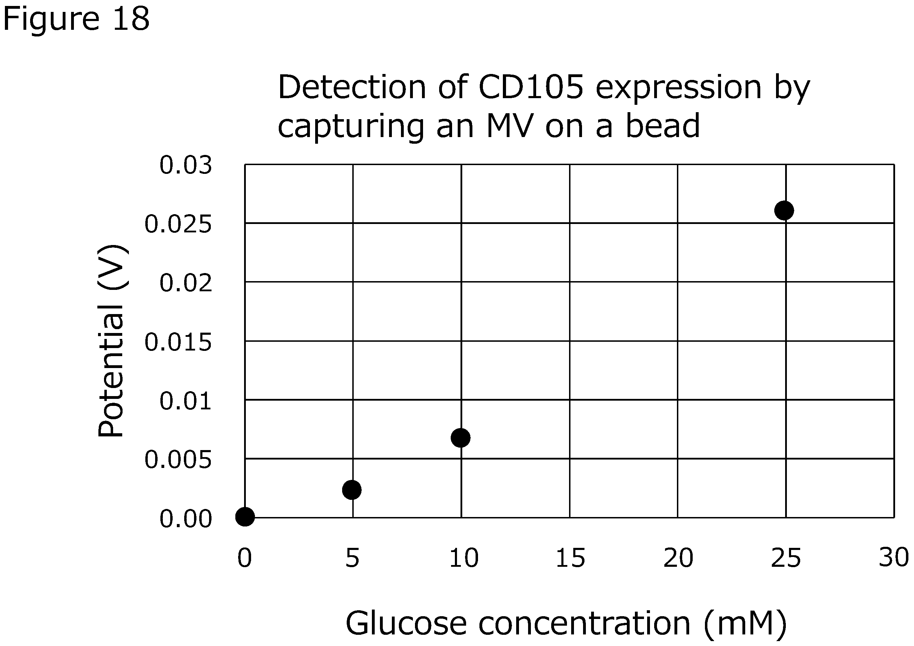

Figure 18 shows the relationship between the output potential change of the ISFET and the glucose concentration at 40 minutes. As the glucose concentration increased, the output potential increased, and it was confirmed that the result was based on the reaction of the enzyme bound to CD105. Further, as compared with Figure 16, where the output potential change in a liquid-phase reaction was 10 mV when the glucose concentration was 25 mM, the output potential change was as large as 26 mV by collecting MVs in the ISFET gate portion using beads and then allowing an enzymatic reaction to occur.

### Example 8: Measurement of the expression level of a protein on a biological vesicle captured by an oleyl group

This example was intended to examine a method for capturing an MV shown in Example 7 efficiently in the further vicinity of the gate portion of the ISFET.

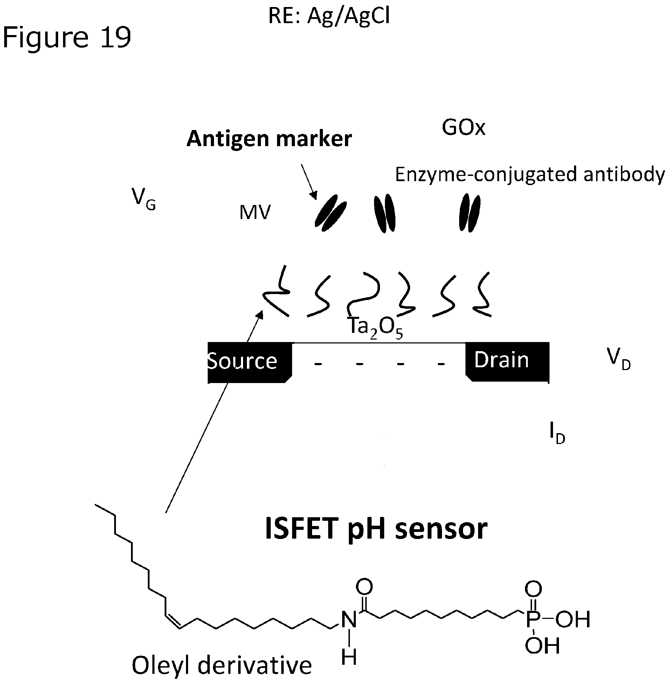

Specifically, the molecule having an oleyl group prepared in Example 2 was immobilized on the gate surface of the ISFET, and an MV was to be captured in the further vicinity of the gate portion of the ISFET (Figure 19). The oleyl group has a high affinity for the MV lipid membrane, is directly fused with the lipid membrane, and can capture the MV on the gate surface. An oleyl group was immobilized by a coupling reaction of a hydroxyl group of a gate oxide film and a phosphonic acid group of an oleyl derivative. In a volume of 20 µL of 1 mM oleyl derivative dissolved in isopropyl alcohol (IPA) was added dropwise to the ISFET equipped with a chamber, and the chamber was air-dried for 1 hour and then completely dried with a nitrogen gas. The chamber was annealed in an oven at 120°C for 1 hour and rinsed three times with 200 µL of IPA.

The MV solution, an anti-mouse IgG secondary antibody (goat) conjugated with an enzyme glucose oxidase (Glucose Oxidase Conjugation Kit-Lightning-Link, Goat anti-Mouse IgG H&L; Abcam), and an anti-human CD105 primary antibody (mouse IgG) (purified anti-human CD105; BioLegend) were mixed, and the mixture was left to stand in a refrigerator for 30 minutes. After centrifugation under a condition of 20,000 g, 4°C, and 30 minutes, the supernatant was discarded, and the precipitates were dissolved in a phosphate buffer solution.

In a volume of 100 µL of MV solution was poured into the ISFET chamber in which an oleyl derivative was immobilized and left to stand at room temperature for 1 hour to immobilize the MV on the ISFET. The supernatant was discarded and replaced with a phosphate buffer solution. Sequentially, a phosphate buffer solution was poured onto the ISFET to start measurement of the potential, a glucose solution (5 mM, 10 mM, or 25 mM) or a phosphate buffer solution was added, and the potential response was recorded.

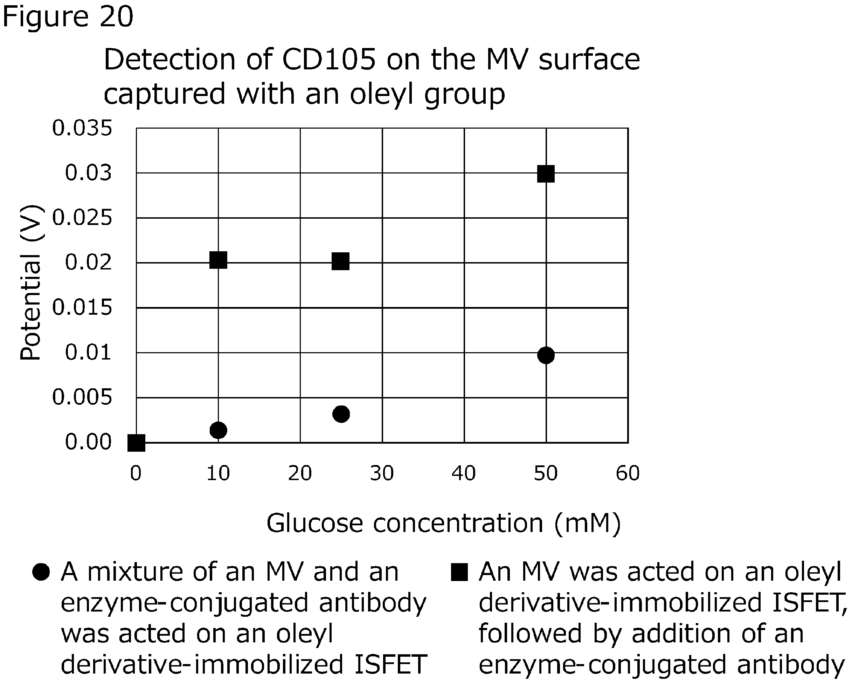

Figure 20 shows the relationship between the output potential change of the ISFET and the glucose concentration at 40 minutes. As the glucose concentration increased, the output potential increased, and it was confirmed that the result was based on the reaction of the enzyme bound to CD105. The data of ■ in the figure are the results obtained after an MV was first allowed to act on the ISFET on which an oleyl derivative was immobilized to capture the MV at the gate, and then an enzyme-conjugated antibody was added. The data of ● are the results obtained after an MV, an antibody, and an enzyme-conjugated antibody were first allowed to react in the liquid phase to form an antibody-enzyme complex on the MV surface and then the MV was captured by the oleyl group on the gate surface.

Both data showed the output potential changed dependently on the glucose concentration, indicating that CD105 on the MV surface could be detected. Of note, the difference in the output potential size was considered to represent a difference in non-specific adsorption due to the difference in the detection protocols.

### [Industrial Applicability]

Given that a body fluid-derived sample holding a plurality of target marker molecules on the surface thereof is solidified on a support in the detecting or measuring technique of the present invention, a plurality of target marker molecules are captured on one support as a result. A pH change or an optical change is generated individually for each of the target marker molecules, and the target marker molecules in the body fluid-derived sample can be detected or measured by detecting or measuring these changes. Further, by capturing one biological vesicle on one sensor on a 1:1 basis and integrating such sensors in a substrate at high density, the number of biological vesicles with a high expression level of the target marker molecule can be counted, and the proportions of biological vesicles with a high expression level of target marker molecules and biological vesicles with a normal expression level can be obtained with high precision. The quantitativity and efficiency of liquid biopsy using a body fluid-derived sample can be improved substantially by using this technique or device.

## Claims

1. A method for detecting or measuring target marker molecule(s) in a body fluid-derived sample on the basis of a pH change or an optical change, the method comprising
a step of capturing a body fluid-derived sample containing target marker molecule(s) on a support,
a step of binding the captured sample to an identification substance that specifically recognizes the target marker molecule(s) in the sample, and
a step of detecting or measuring a pH change or an optical change generated by the identification substance bound to the target marker molecule(s).

2. The method according to claim 1, wherein a plurality of target marker molecules are contained in a single body fluid-derived sample.

3. The method according to claim 2, wherein a step of binding one kind of identification substance to a single body fluid-derived sample and a step of detecting or measuring a pH change or an optical change resulting therefrom are repeated a plurality of times.

4. The method according to claim 3, wherein an enzyme bound to a plurality of identification substances that are used in the steps repeated a plurality of times is one kind of the enzyme.

5. The method according to claim 2, wherein a step of binding a plurality of the identification substances to a single body fluid-derived sample and then detecting or measuring a pH change or an optical change resulting from each identification substances is repeated a plurality of times.

6. The method according to claim 5, wherein a different enzyme is bound to each of a plurality of different identification substances.

7. The method according to any one of claims 1 to 6, wherein the identification substance is an antibody that specifically binds to the target marker molecule(s).

8. The method according to any one of claims 1 to 7, wherein the body fluid-derived sample is a cell or a biological vesicle.

9. The method according to claim 8, wherein the biological vesicle is an exosome or an extracellular vesicle.

10. The method according to any one of claims 1 to 9, wherein the pH change or the optical change is generated by a substrate-enzyme reaction.

11. A device for detecting or measuring target marker molecule(s) in a body fluid-derived sample, the device comprising
a support for capturing the body fluid-derived sample, and
a semiconductor sensor for detecting or measuring a pH change or an optical change generated by an identification substance that specifically recognizes the target marker molecule(s) in the body fluid-derived sample.

12. The device according to claim 11, wherein the semiconductor sensor and the support are in the same structure.

13. The device according to claim 11 or 12, wherein the semiconductor sensor is a pH sensor selected from an ISFET, an extended-gate FET, and a floating-gate FET or a photosensor selected from a photodiode, a phototransistor, a CCD, and a CMOS image sensor.

14. The device according to any one of claims 11 to 13, wherein the support is selected from a pH sensitive film composed of tantalum oxide, iridium oxide, silicon nitride, hafnium oxide, or the like, a glass, a simple silicone substance, a carbon material such as carbon nanotube or graphite, a two-dimensional material such as graphene or molybdenum sulfide, and GaAs.

15. The device according to any one of claims 11 to 14, wherein the surface of the support is modified to capture a body fluid-derived sample.

16. The device according to claim 15, wherein the modification is performed by applying a surface modifier selected from an antibody, a cationic polymer, an extracellular matrix protein, and a hetero molecule having a lipid and a phosphoric acid group, a phosphonic acid group, or a silanol group.

17. The device according to any one of claims 11 to 16, wherein the semiconductor sensor electrically detects or measures a pH change or an optical change generated by a substrate-enzyme reaction.

18. A method for modifying a support for capturing a sample in a body fluid by applying a surface modifier selected from an antibody, a cationic polymer, an extracellular matrix protein, and a hetero molecule having a lipid and a phosphoric acid group, a phosphonic acid group, or a silanol group, and a sulfur-containing self-assembled film on the surface of the support.

19. The method according to claim 18, for selectively capturing a cell or a biological vesicle in a body fluid.

20. The method according to claim 19, wherein the biological vesicle is an exosome or an extracellular vesicle.

21. A substance for capturing a body fluid-derived sample on a support, the substance is composed of (1) a site to bind to a body fluid-derived sample and (2) a site to bind to a support material.

22. The substance according to claim 21, wherein the body fluid-derived sample is a cell or a biological vesicle.

23. The substance according to claim 22, wherein (1) the site to bind to a body fluid-derived sample is a hydrocarbon chain or a long-chain fatty acid that binds to the lipid membrane of a cell or a biological vesicle.

24. The substance according to claim 23, which is a hydrocarbon chain or a long-chain fatty acid that has four or more carbon atoms.

25. The substance according to any one of claims 21 to 24, wherein (2) the site to bind to a support material is composed of a silane compound, a phosphoric acid group, a phosphonic acid group, a thiol group, or a disulfide group.

26. The substance according to claim 21, wherein the substance is a compound represented by the following:

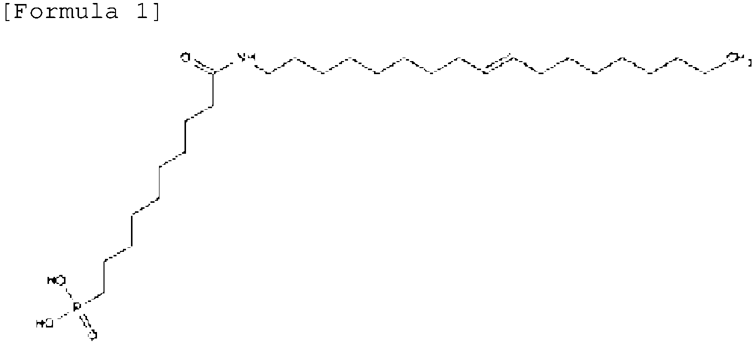

27. A method for analyzing the expression level of target marker molecule(s), the method comprising
capturing a single body fluid-derived sample on each of a plurality of sensing regions of a semiconductor sensor in a device,
binding an identification substance that specifically recognizes a target marker molecule in the sample,
inducing a pH change or an optical change in the vicinity of the sensing region by introducing a substrate,
digitally counting the number of body fluid-derived samples with a high expression level of the target marker molecule based on the pH change or the optical change, and
counting the number of semiconductor sensors that have captured body fluid-derived samples with a high expression level of the target marker molecule.
